# EUROPÄISCHE PATENTSCHRIFT

(11) **EP 1 100 565 B1**
(45) Veröffentlichungstag und Bekanntmachung des Hinweises auf die Patenterteilung: **15.12.2004**
(21) Anmeldenummer: 00947760.5
(22) Anmeldetag: 23.05.2000
(51) Int. Cl.: A61M 5/142

(54) **IMPLANTIERBARE INFUSIONSPUMPE MIT FÜLLSTANDSMESSUNG**
IMPLANTABLE INFUSION PUMP WITH LEVEL MEASURING
POMPE DE PERFUSION IMPLANTABLE, A MOYENS INDICATEURS DE NIVEAU

(30) Priorität: 26.05.1999 DE 19924031
(43) Veröffentlichungstag der Anmeldung: 23.05.2001
(73) Patentinhaber: Codman Neuro Sciences Sàrl, 2400 Le Locle (CH)
(72) Erfinder: WIELAND, Manfred, D-24146 Kiel (DE); ZACHARIAS, Volker, D-24106 Kiel (DE); WIERZOCH, Jan, D-25436 Moorregge (DE)
(74) Vertreter: Bardehle, Heinz, Dipl.-Ing.
(86) Internationale Anmeldenummer: PCT/DE2000/001631
(87) Internationale Veröffentlichungsnummer: WO 2000/072899

(56) Entgegenhaltungen:
- DE-A- 2 604 113
- DE-A- 3 520 782
- DE-C- 19 624 215
- US-A- 4 002 996
- US-A- 4 354 506

## Beschreibung

Die Erfindung betrifft eine implantierbare Infusionspumpe gemäß dem Oberbegriff des Anspruchs 1.

Derartige Infusionspumpen, wie sie beispielsweise aus der DE 26 04 113 C2 bekannt sind, werden Patienten implantiert, die über einen langen Zeitraum kontinuierlich mit einem Medikament zu versorgen sind, beispielsweise Schmerzpatienten oder Spastikern.

Aus der DE 196 24 215 C1 ist es bekannt, eine Infusionspumpe mit einen elektromagnetischen Schwingkreis zu versehen, dessen eines Element zur Füllstandsmessung mechanisch mit dem Boden des Balges verbunden ist.

Der Erfindung liegt die Aufgabe zugrunde, eine implantierbare Infusionspumpe zu schaffen, die auf Grund besonders einfacher Weise die Erfassung des Füllstandes erlaubt.

Erfindungsgemäß wird diese Aufgabe gelöst durch die Merkmale des kennzeichnenden Teils von Anspruch 1.

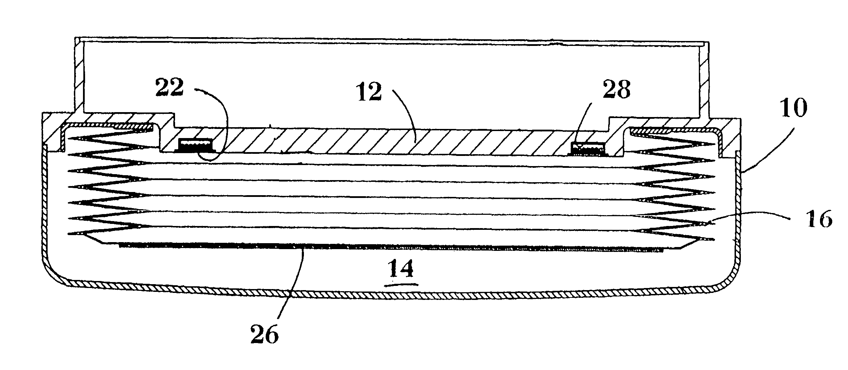

Die Erfindung wird im folgenden anhand einer Zeichnung erläutert. Dabei zeigt die einzige Figur eine Schnittdarstellung des unteren Teiles einer derartigen Infusionspumpe.

Die Infusionspumpe weist ein Gehäuse 10, eine Grundplatte 12, einen Treibmittelraum 14, ein das abzugebende Medikament aufnehmenden Balg 16 und eine zu einem in dem Körper des Patienten verlegten Katheter führende Drosselstrecke (nicht gezeigt) auf. In einer Vertiefung in der zu dem Boden 26 des Balges 16 weisenden Fläche der Grundplatte 12 ist eine Vertiefung angeordnet, die eine Flachspule 22 aufnimmt. Diese Flachspule 22 bildet gemeinsam mit einem - nicht gezeigten - Kondensator einen Schwingkreis. Die Flachspule 22 ist zu der Grundplatte 12 durch ein Element 28 elektromagnetisch abgeschirmt, zu dem Medikamentenraum ist die Spule durch ein für elektromagnetische Energie transparente Abdeckung abgedeckt.

Bei dem durch die Spule 22 und den Kondensator gebildeten Schwingkreis ist die Flachspule 22 das frequenzbestimmende Element. Diese erzeugt bei Erregung ein primäres elektromagnetisches Feld mit großer Apertur, das den Boden 26 des Balges 16 durchflutet und in diesem Wirbelströme induziert. Diese Wirbelströme erzeugen ihrerseits ein sekundäres magnetisches Feld, das über das Induktionsgesetz mit dem Primärfeld verkoppelt ist. Diese Kopplung bewirkt eine Änderung der Induktivität der Flachspule 22 und damit eine Verschiebung der Resonanzfrequenz des Schwingkreises.

Die rückwärtige Abschirmung des Spulenfeldes durch das MU-Metall-Element 28 bewirkt zum einen, daß die Wirbelströme in der Grundplatte 12 erheblich reduziert werden. Zum anderen wird die Phase des nach oben, also von dem Balg weg weisenden Feldes beeinflußt, so daß eine unterstützende Wirkung in Richtung auf den Balg resultiert.

Die Induktivitätsänderung geschieht nicht durch die Änderung der Permeabilität, also der magnetischen Leitfähigkeit der Spulenumgebung, sondern durch die Kopplung der Felder.

Der Meßeffekt kann durch Aufbringen einer dünnen Schicht aus einem guten elektrischen Leiter (beispielsweise Kupfer) auf die Unterseite des Balges verstärkt werden.

Ein Kippen des Bodens 26 des Balges 16 relativ zu der Grundplatte 12 ist ohne erhebliche Bedeutung, da sich die durch das Kippen bedingten unterschiedlichen Abstände weithin kompensieren.

Von Vorteil ist es weiter, daß die Meßgenauigkeit mit abnehmendem Abstand des Bodens 26 des Balges 16, also mit geringer werdendem Volumen des Balges 16 zunehmend größer wird.

## Patentansprüche

1. Implantierbare Infusionspumpe mit einem Gehäuse (10), einer Grundplatte (12), einem Treibmittelraum (14), einem das abzugebende Medikament aufnehmenden Balg (16) und einer mit dem Raum des Balges (16) kommunizierenden und zu einem in dem Körper des Patienten verlegten Katheter führenden Drosselstrecke, und einem durch eine Spule (22) und einen Kondensator gebildeten Schwingkreis, dessen Resonanzfrequenz von dem Abstand des Bodens (26) des Balges (16) von der Grundplatte (12) bestimmt wird,
**dadurch gekennzeichnet, daß**
die Spule (22) als Flachspule ausgebildet und an oder in der zu dem Boden (26) des Balges (16) weisenden Fläche der Grundplatte (12) angeordnet ist, wobei die Spule (22) bei Erregung ein primäres elektromagnetisches Feld erzeugt das den Boden (26) des Balges (16) durchflutet und in diesem Wirbelströme induziert, die ihrerseits ein sekundäres magnetisches Feld erzeugen, und wobei eine Verschiebung der Resonanzfrequenz durch die Kopplung des primären und des sekundären magnetischen Felds bewirkt wird.

2. Implantierbare Infusionspumpe nach Anspruch 1, gegen die Grundplatte (12) elektromagnetisch abgeschirmt ist.

3. Implantierbare Infusionspumpe nach Anspruch 1, **dadurch gekennzeichnet, daß** die Unterseite des Bodens des Balges mit einer dünnen Schicht eines guten elektrischen Leiters belegt ist.

## Claims

1. Implantable infusion pump with a housing (10), a ground plate (12), a propellant means space (14), a bellows (16), receiving the medicament to be provided and a restrictor line communicating with the space of the bellows (16) and leading to a catheter, which is laid within the body of the patient and an oscillator formed by a coil (22) and a capacitor, which resonance frequency is determined by the distance of the floor (26) of the bellows (16) to the ground plate (12),
**characterized in that**
the coil (22) is provided as a flat coil and arranged at or within a surface of the ground plate (12), which shows to the floor (26) of the bellows (16), wherein the coil (22) generates a primary electromagnetic field during excitement, which floods through the floor (26) of the bellows (16) and induces eddy currents therein, which on their part generate a secondary magnetic field, and wherein a shift of the resonance frequency is caused by the coupling of the primary and the secondary magnetic field.

2. Implantable infusion pump according to claim 1, wherein the ground plate (12) is electromagnetically shielded.

3. Implantable infusion pump according to claim 1, **characterized in that** the lower side of the floor of the bellows is covered with a thin layer of a good electrical conductor.

## Revendications

1. Pompe à infusion implantable avec un boîtier (10), une plaque de base (12), une chambre pour fluide d'entraînement (14), un soufflet (16) recevant le médicament à administrer, et une partie en étranglement communiquant avec le volume du soufflet (16) et conduisant au cathéter placé dans le corps du patient, et un circuit oscillant, formé d'un bobinage (22) et d'un condensateur, dont la fréquence de résonance est déterminée par l'écartement entre le fond (26) du soufflet (16) et la plaque de base (12),
**caractérisée en ce que**
le bobinage (22) est constitué d'un bobinage plat et il est monté sur ou dans la face de la plaque de base (12) tournée vers le fond (26) du soufflet (16), le bobinage (22) produisant par excitation un champ électromagnétique primaire qui traverse le fond (26) du soufflet (16) et induit dans celui-ci des courants de Foucault qui de leur côté produisent un champ magnétique secondaire, et un décalage de la fréquence de résonance étant produit par le couplage des champs magnétiques primaire et secondaire.

2. Pompe à infusion implantable selon la revendication 1, avec contre la plaque de base (12) un blindage électromagnétique.

3. Pompe à infusion implantable selon la revendication 1, **caractérisée en ce que** la sous-face du fond du soufflet est recouverte d'une mince couche d'un bon conducteur électrique.
